# EUROPEAN PATENT APPLICATION

(11) **EP 3 127 909 A1**
(43) Date of publication of application: **08.02.2017**
(21) Application number: 16187004.3
(22) Date of filing: 03.07.2013
(51) Int. Cl.: C07D 498/22

(54) **CRYSTALLINE FORMS OF RIFAXIMIN AND THEIR PREPARATION IN THE PRESENCE OF AMINO ACIDS**

(30) Priority: 06.07.2012 IT BO20120368
(62) Divisional of application: 13739813.7
(71) Applicant: ALFA WASSERMANN S.p.A., 65020 Alanno (PE) (IT)
(72) Inventor: VISCOMI, Giuseppe Claudio, 40133 Bologna (IT); CHELAZZI, Laura, 40133 Bologna (IT); GREPIONI, Fabrizia, 40133 Bologna (IT); BRAGA, Dario, 40133 Bologna (IT); KINDT, Maddalena, 40133 Bologna (IT)
(74) Representative: Hiebl, Inge Elisabeth

(57) **Abstract**

The object of the present invention concerns rifaximin compositions comprising amino acids, characterized in that they increase rifaximin solubility in aqueous solutions.

Rifaximin compositions comprising amino acids are useful in the treatment of disease wherein amino acids and rifaximin are efficacious.

The present invention relates to pharmaceutical compositions comprising rifaximin or one of the pharmaceutically acceptable salts thereof and one or more amino acid(s), wherein the molar ratio between the amino acid(s) and rifaximin is comprised between 1:1 and 10:1, preferably between 1:1 and 5:1, together with pharmaceutically acceptable excipients. Moreover, the present invention relates to rifaximin crystals characterized in that they are obtained by means of a process comprising:
a) dissolution of the compositions of rifaximin and amino acids, wherein amino acids and rifaximin are in a molar ratio comprised between 1:1 and 10:1 according to the above compositions, in solutions formed by ethanol/water, in a volumetric ratio comprised between 1:1 and 1:10 (v/v);
b) evaporation of the solution obtained in step a) at temperatures comprised between room temperature and 40°C, in a time period comprised between 1 and 10 days;
wherein the resulting crystals have monoclinic space group *P*2₁ and cell parameters comprised in the ranges: a: 13.7(1)-13.8(1) *Å*; b: 19.7(1)-19.9 *(1)Å;* c: 16.4(6)-16.6(6) A; β: 92.1(1)-91.9(1) deg.

## Description

### Field of the invention

Rifaximin (INN; see The Merck Index, XIII ed., 8304) is a semi-synthetic non-aminoglycoside derived from rifamycin. More precisely, it is a pyrido-imidazo rifaximin, described and claimed in the Italian patent IT 1154655, whereas the European patent EP 0161534 describes a process for its production starting from rifamycin O.

From the chemical point of view, rifaximin is (S-S, 16Z, 18E, 20S, 21 S, 22R, 23R, 24R, 25S, 26S, 27S, 28E)-5, 6, 21, 23, 25-pentahydroxy-27-methoxy-2, 4, 11, 16, 20, 22, 24, 26-octamethyl-2,7-(epoxy pentadeca- [1,11,13] trienimino)-benzofuro [4,5-e]-pyrido[1,2-(alpha)]- benzimidazole-1,15(2H) dione, 25-acetate, and is represented in Figure 1.

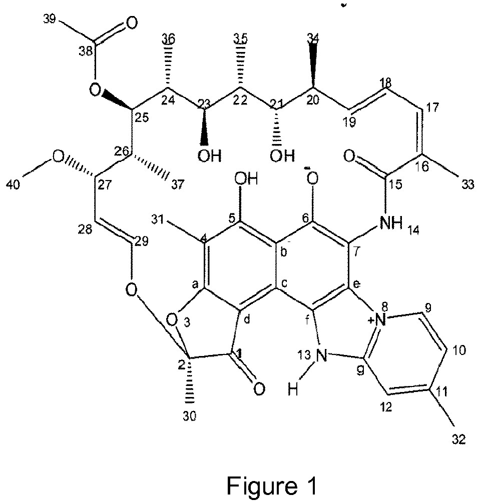

Rifaximin is also currently sold with the trademark Normix^{®}, Rifacol^{®} and Xifaxan^{®}.

Rifaximin is an antibiotic usually used for local action with a broad spectrum of action against Gram-positive and Gram-negative bacteria and aerobic and anaerobic organisms. Rifaximin has an excellent safety profile and it is characterized for a non- systemic absorption.

Rifaximin is used for the treatment of bowel infections caused by non-enteroinvasive bacteria, traveler's diarrhea, enteritis, dysentery, bowel inflammations such as, for instance, Crohn's disease (CD), ulcerous recto-colitis, irritable bowel syndromes (IBS), paucities, small intestinal bacterial overgrowth (SIBO), diverticular syndromes; pathologies directly or indirectly deriving from bowel infections, such as for instance hepatic encephalopathy, or which can be used in the pre- and post-operative prophylaxis of bowel infections.

US 4,557,866 describes a new process for the synthesis of pyrido-imidazo rifaximins comprising the reaction of rifamycin O with 4-methyl-2-aminopiridine.

EP 1557421 B1, EP 1676847 B1, EP 1676848 B1 and US 7,045,620 B2 describe polymorphic forms of rifaximin (INN), called rifaximin α, rifaximin β, and a poorly crystalline form called rifaximin γ. These forms can be obtained by hot-dissolving the raw rifaximin in ethyl alcohol and by inducing subsequent crystallization of the product by addition of water at a given temperature and for a fixed time. The crystallization is then followed by a drying step carried out under controlled conditions until a predefined water content is obtained, and the X-ray diffraction profile corresponds to one observed for the aforesaid rifaximin forms.

These patents also describe processes for the transformation from a polymorphic form to another ones, such as the obtainment of polymorph α by dehydration of polymorph β or of polymorph γ; the obtainment of polymorph γ starting from polymorph α and the preparation of polymorph β by hydration of polymorph α.

US 7,906,542 B2 describes pharmaceutical compositions comprising polymorphic forms of rifaximin α, β and γ.

EP 1682556 A2 describes polymorphic forms of rifaximin α, β and γ characterized in that they have quite different in vivo absorptions and dissolution profiles.

US 7,915,275 B2 describes the use of pharmaceutical compositions comprising polymorphic forms of rifaximin α, β and γ for the treatment of bowel infections.

WO 2008/155728 describes a process for obtaining amorphous rifaximin, wherein said process is carried out by hot-dissolving raw rifaximin in absolute ethyl alcohol and then collecting after precipitation by cooling rifaximin under amorphous form.

Amorphous forms of rifaximin and processes for their obtainment are described in US 2009/312357 and US 2009/0082558, in particular US 2009/0082558 describes that amorphous rifaximin is obtained after precipitating by addition of water to a rifaximin solution in organic solvent.

WO 2009/108730 describes polymorphic forms of rifaximin (form ζ, form γ-1 (ζ), form α-dry, form η, form 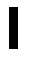 , form β-1, form β-2, form ε-dry), salts, hydrates and amorphous rifaximin, their use in the preparation of pharmaceutical compositions and therapeutic methods related to their use.

WO 2011/153444 describes polymorphic forms of rifaximin k and θ and WO 2011/156897 describes polymorphic forms of rifaximin called APO-1 and APO-2.

WO 2006/094662 describes polymorphic forms δ and ε of rifaximin useful in the preparation of pharmaceutical forms for oral and topical use. Said forms are obtained by means of processes comprising hot dissolution of raw rifaximin in ethyl alcohol, then addition of water at predetermined temperatures and for predetermined time periods, then drying under vacuum.

Viscomi et al. in CrystEngComm, 2008, 10, 1074-1081 describes the process for the preparation of polymorphic forms of rifaximin and their chemical, physical and biological characteristics.

Bacchi A. et al. in New Journal of Chemistry (2008), 32; 10; 1725-1735, describe the obtainment of crystals of tetra-hydrated rifaximin β with a water weight content corresponding to 8.4% (w/w), obtained by slowly evaporating water/ethanol solution of rifaximin at room temperature.

Rifaximin is a substantially water-insoluble molecule, and organic solvents are necessary to be added for increasing its solubility in aqueous solutions. Organic solvents are hardly acceptable in the preparation of substances for pharmaceutical use, and their use requires severe controls of the residual solvents in the final products.

Rifaximin water solubility can be varied within limited concentration ranges by selecting suitable polymorphic or amorphous forms. For example, WO 2005/044823 states that rifaximin polymorph α is substantially insoluble, whereas WO 2011/107970 states that an amorphous form of rifaximin obtained by means of spray-drying has a solubility of about 40 µg/ml after thirty minutes in aqueous solution, but this form is not stable and the solubility decrease in the time and after two hours it is about 9 µg/ml.

As described by Viscomi et al. in CrystEngComm, 2008, 10, 1074-1081, rifaximin solubility in suspension the presence of solid rifaximin may vary during the time according to possible transformation processes in more stable crystalline forms. In particular, it is described that also in case of substantially amorphous rifaximin polymorphs, solubility decreases in time until it coincides with the values obtainable with the more stable crystalline forms.

Rifaximin is also a local-action antibiotic, and the in-situ bioavailability of pharmaceutical compositions providing for increased available and local rifaximin concentrations (e.g. in physiological fluids such as e.g. gastric and intestinal fluids) is useful for treating all pathologies wherein increased rifaximin concentration can provide higher therapeutic efficacy.

There is a need in the art for formulation of rifaximin with increased rifaximin solubility in aqueous solution for obtaining increased rifaximin concentrations, higher and stable in time in respect to those obtainable by the prior art.

There is also a need to have rifaximin pharmaceutical composition also comprising amino acids for the treatment of all the disease wherein the aminoacids are efficacious. There is also a need to associate to the antibiotic effect of rifaximin the effect of the amino for the treatment of hepatic disease and debilitated disease.

There is also a need to obtain compositions providing increased rifaximin concentrations at room temperature, to be used directly in pharmaceutical preparations, for instance in form of tablets or clear solutions replacing granulate in cloudy suspensions, which are accepted with difficulty by patients, or in compositions for vaginal or rectal use. Preferably compositions having rifaximin concentrations higher than 3 µg/ml at room temperature should be obtained.

It is also convenient to have rifaximin solutions with increased rifaximin concentration for reducing the volumes of the solution in industrial preparations without the addition of large volumes of organic solvents.

In particular, in the treatment of some bowel infections, it would be useful having available gastroresistant compositions promoting the release of high concentrations of rifaximin in the intestine.

In the prior art, rifaximin can be obtained in powder, in raw form, in polymorphic or amorphous forms.

The information concerning the crystalline characteristics of rifaximin derivatives available in the prior art has been obtained by means of the X-ray powder diffraction technique. The available diffractograms are the result of the contribution of several micro-crystals forming the powder; they can show diffraction signals which are often broadened and have a non-constant intensity, even analyzing the same sample, since they can be influenced by several factors, such as, for instance, the size and morphology of the crystallites and their distribution in the sample holder. Therefore, the univocal attribution to a settled phase of a water content as well as of an exact proportion of possibly present solvates and/or hydrates by means of X-ray powder diffraction can be rather difficult.

Generally, the size and patterns of crystals can have an influence on some properties of the powder of an active principle. For example, Kiang Y.H et al. describe in Int. J. Pharm. 368 (2009, 76) that compressibility and flowability are some of the properties which can be related to crystal morphology and that these properties have an influence on the preparation of finished compositions in solid form.

Vippagunta S.R. et al. in Adv. Drug. Del. Rev. 48 (2001), 3-26, state the relevance of controlling the crystalline forms of an active principle during the various stages of its development, because each phase change due to interconversion of the polymorphs, to solvation processes, to hydrate formation and to change of crystallinity degree can vary the bioavailability of the drug.

The correlation between solid structure and pharmacologically useful properties, such as e.g. bioavailability, is now ascertained. In fact, for giving their approval to the commercialization of drugs, Health Authorities require suitable analytical techniques for identifying the crystalline structure of the active principle, as well as production processes of the finished product for obtaining consistent amounts of the specific polymorphic forms.

Therefore, the availability of crystals showing high quality, purity and suitable size is critical and quite useful for using them as analytical standards, for determining single polymorphs possibly present in mixtures, for determining properties related to the molecule pattern in a crystal, and for determining the possible presence and number of molecules of water or another solvent in the structure.

The availability of crystals having a size such that X-ray diffraction can be carried out on a single crystal is highly relevant; the presence of said polymorphs in complex mixtures and also the exact water content of a polymorph could be determined thanks to the information provided by such technique.

A better knowledge of the crystalline structure is also relevant for modifying the production processes in order to obtain compounds with reproducible crystallinity, thus guaranteeing the presence of crystallinity-related properties in the preparation of reproducible pharmaceutical compositions.

It is well known that the presence or the absence of water molecules in specific crystalline positions can have an influence on the position of the peaks in a powder diffractogram and, in the case of rifaximin, knowing such positions would allow a better interpretation of these diffractograms.

For example, a quantitative characterization of an amorphous compound is difficult because it does not give specific signals, but is detectable only by observing a raising diffractogram baseline. Therefore, the availability of crystals with determined crystallographic parameters would allow the determination of the amount of amorphous substance in a mixture, when the amorphous form goes with the known crystalline forms.

An object of the present invention is represented by compositions deriving from the association of rifaximin and amino acids, wherein amino acids and rifaximin are in a molar ratio comprised between 1:1 and 10:1, said compositions allowing the obtainment of rifaximin crystals and being suitable to be used for the preparation of pharmaceutical compositions.

An object of the present invention is represented by rifaximin crystals obtained when the compositions of rifaximin and amino acids are solubilized in aqueous solutions, then undergoing controlled drying processes. Said crystals are useful as analytical standards. It is also an object of the present invention the use of amino acids for obtaining increased rifaximin concentrations if compared to the state of the art.

In particular, the effect of amino acids is such that it provides rifaximin concentrations in water higher than 3 µg/ml at room temperature and higher than 7 µg/ml at 37°C.

The present invention also shows that amino acids have a synergic effect together with organic solvents in increasing rifaximin solubility in aqueous solutions containing low percentages of organic solvents.

The use of amino acids, object of the present invention, therefore allows the preparation of compositions with rifaximin, the obtainment of rifaximin crystals, the increasing of rifaximin concentration in aqueous solutions and the preparation of pharmaceutical compositions.

### Summary of invention

The present invention relates to pharmaceutical compositions comprising rifaximin or one of the pharmaceutically acceptable salts thereof and one or more amino acid(s), wherein the molar ratio between the amino acid(s) and rifaximin is comprised between 1:1 and 10:1, preferably between 1:1 and 5:1, together with pharmaceutically acceptable excipients, which provide increased rifaximin solubility in aqueous solution.

The invention further relates to a process for preparing said pharmaceutical compositions comprising the steps
- mixing rifaximin and amino acids;
- adding the excipients and mixing the final mixture in a V mixer for a time between 10 and 30 minutes and
- granulating in a roller compactor.

The invention further relates to the use of an amino acid for obtaining a pharmaceutical composition having a rifaximin concentration from 4.5 µg/ml to 60 µg/ml.

The invention further relates to rifaximin crystals characterized in that they are obtained by means of a process comprising:
a) dissolution of the compositions of rifaximin and amino acids, wherein amino acids and rifaximin are in a molar ratio comprised between 1:1 and 10:1 according to the above compositions, in solutions formed by ethanol/water, in a volumetric ratio comprised between 1:1 and 1:10 (v/v);
b) evaporation of the solution obtained in step a) at temperatures comprised between room temperature and 40°C, in a time period comprised between 1 and 10 days;
wherein the resulting crystals have monoclinic space group *P*2₁ and cell parameters comprised in the ranges: a: 13.7(1)-13.8(1) *Å*; b: 19.7(1)-19.9 *(1)Å*; c: 16.4(6)-16.6(6) *Å*; β: 92.1(1)-91.9(1) deg.

The invention further relates to a process for the production of rifaximin crystals, characterized in that it comprises:
a) dissolution of the compositions of rifaximin and amino acids, wherein amino acids and rifaximin are in a molar ratio comprised between 1:1 and 10:1 according to the above compositions, in solutions formed by ethanol/water, in a volumetric ratio comprised between 1:1 and 1:10 (v/v);
b) evaporation of the solution obtained in step a) at temperatures comprised between room temperature and 40°C, in a time period comprised between 1 and 10 days;
wherein the resulting crystals are characterized by monoclinic space group *P*2₁ and cell parameters comprised in the ranges: a: 13.7(1)-13.8(1) *Å*; b: 19.7(1)-19.9 *(1)Å*; c: 16.4(6)-16.6(6) *Å*; β: 92.1(1)-91.9(1) deg.

The invention further relates to a process for the production of rifaximin crystals, characterized in that it comprises:
a) dissolution of the compositions of rifaximin and amino acids, wherein amino acids and rifaximin are in a molar ratio comprised between 1:1 and 10:1 according to the above compositions, in solutions formed by ethanol/water, in a volumetric ratio comprised between 1:1 and 1:10 (v/v);
b) evaporation of the solution obtained in step a) at temperatures comprised between room temperature and 40°C, in a time period comprised between 1 and 10 days, in the presence of dehydrating agents;
wherein the resulting crystals have monoclinic space group *P*2₁ and cell parameters comprised in the ranges: a: 14.2(1)-14.5(1) *Å;* b: 19.7(1)-20.1(1) *Å*; c: 16.1(1)-16.2(1)*Å*; β: 108.7(1)-111.4(1)deg.

The invention further relates to the use of amino acids and rifaximin in a molar ratio comprised between 1:1 and 10:1 in order to obtain rifaximin crystals, characterized by monoclinic space group *P*2₁ and cell parameters comprised in the ranges: a: 13.7(1)-13.8(1) *Å*; b: 19.7(1)-19.9 *(1) Å*; c: 16.4(6)-16.6(6) *Å*; β: 92.1(1)-91.9(1) deg.

The invention further relates to the use of amino acids and rifaximin in a molar ratio comprised between 1:1 and 10:1 in order to obtain rifaximin crystals, characterized by monoclinic space group *P*2₁ and cell parameters comprised in the ranges: a: 14.2(1)-14.5(1) *Å*; b: 19.7(1)-20.1(1) *Å*; c: 16.1(1)-16.2(1) *Å*; β: 108.7(1)-111.4(1) deg.

The crystalline form of rifaximin in the pharmaceutical compositions according to the present invention may be selected from
i) crystals having monoclinic space group P2₁ and cell parameters comprised in the ranges: a: 13.7(1)-13.8(1) *Å*; b: 19.7(1)-19.9 *(1) Å*; c: 16.4(6)-16.6(6) *Å*; β: 92.1(1)-91.9(1) deg.,
ii) crystals having the features of i) and having 3 or 4.5 water molecules for each rifaximin molecule,
iii) crystals having monoclinic space group P2₁ and cell parameters comprised in the ranges: a: 14.2(1)-14.5(1) *Å*; b: 19.7(1)-20.1(1) *Å*; c: 16.1(1)-16.2(1) *Å*; β: 108.7(1)-111.4(1) deg.
iv) crystals having the features of iii) and having zero or 0.5 or 1.5 water molecules for each rifaximin molecule, or
v) rifaximin α, β, γ, δ.

The compositions of the present invention comprise rifaximin in form of hydrate, solvate, polymorphous, amorphous or crystalline form or their mixture.

These compositions are useful for treating and preventing inflammatory and infection diseases susceptible to rifaximin treatment.

The compositions of the invention comprise one or more amino acids wherein amino acids are aliphatic amino acids, aromatic amino acids, basic amino acids, branched amino acids, cyclic amino acids, acid amino acids or amide amino acids, or their mixtures.

In particular the compositions of the invention comprise one or more aromatic or heterocyclic amino acids.

In one particular aspect the composition comprise a mixture of one or more aromatic or heterocyclic amino acids and branched amino acids.

Pharmaceutical compositions of the invention comprise rifaximin in a dosage form from 20 mg to 1200 mg with amino acids in a molar ratio between 1:1 and 10:1, preferably between 1:1 and 5:1, and they can be administered in a dosage range from 20 to 3000 mg per day.

Pharmaceutical compositions of the inventions can be in form of granulates, tablets capsules, cream, ointment, suppository, suspension or solution. They are for human or animal use.

Pharmaceutical compositions of the invention can comprise amino acids and rifaximin in form of powder, homogeneously mixed, or in form of "conglomerate", wherein the term conglomerate indicates rifaximin crystals and amino acids, which are generated to form a more cohesive mass between rifaximin and amino acids.

Conglomerates are obtained when amino acids and rifaximin, in a molar ratio between 1:1 and 10:1, preferably between 1:1 and 5:1, are solubilized in aqueous solutions in the presence of organic solvent, preferably alcohol, in a volumetric ratio between 5% to 25% at a temperature between ambient temperature and boiling temperature and the solvent evaporated.

Another aspect of the invention is pharmaceutical compositions comprising rifaximin conglomerates and acceptable excipients, characterized for providing increased rifaximin solubility with respect to the prior art.

Another aspect of the invention are conglomerates of rifaximin which are characterized by comprising rifaximin crystals.

The rifaximin crystals, obtained by conglomerate are in a form of crystal and in particular in form of single crystal, characterized by defined crystal parameters and water contents.

Another aspect of the invention are the processes for the preparation of the pharmaceutical compositions comprising amino acids and rifaximin, wherein amino acids are in a molar ratio between 1:1 to 10:1 for the preparation of solid forms as tablets, granulates, ointments, cream, suppositories, solution.

Another aspect are amino acids and rifaximin pharmaceutical compositions for treatment or in the prevention of infections and disease, wherein rifaximin and amino acid are efficacious.

A particular aspect of the invention is the composition comprising amino acids and rifaximin for the treatment of traveler's diarrhea, hepatic encephalophathy, infectious diarrhea, diverticular disease, an antibacterial prophylactic prior and post colon surgery, irritable bowel syndrome, Crohn's disease, Clostridium difficile-associated diarrhea, small intestinal overgrowth, traveler's diarrhea prophylaxis, dysentery, pauchitis, peptic ulcer disease, surgical prophylaxis and gastric dyspepsia by administering composition comprising rifaximin and amino acids.

Another aspect are amino acids-rifaximin pharmaceutical compositions comprising at least a branched amino acid beneficial in the treatment of hepatic disease as hepatic encephalopathy and complication of cirrhosis disease.

The pharmaceutical compositions object of the present invention provide increased local concentrations of rifaximin and combine the antibiotic effect of rifaximin with the energetic and nourishing effect of amino acids obtaining a beneficial effect for the patient.

Another aspect is the use of amino acids for increasing rifaximin solubility, also in the presence of small volume of organic solvents.

Moreover, the application comprises the following items:
1. Pharmaceutical composition comprising rifaximin or one of the pharmaceutically acceptable salts thereof and one or more amino acid(s), wherein the molar ratio between the amino acid(s) and rifaximin is comprised between 1:1 and 10:1, preferably between 1:1 and 5:1, together with pharmaceutically acceptable excipients.
2. The pharmaceutical composition according to item 1, wherein the rifaximin is in the crystalline, polymorphous or amorphous form, in the form of a hydrate or solvate and/or in a mixture thereof.
3. The pharmaceutical composition according to item 2, wherein the crystalline form of rifaximin is selected from
   i) crystals having monoclinic space group P2₁ and cell parameters comprised in the ranges:
      a: 13.7(1)-13.8(1) *Å*; b: 19.7(1)-19.9 *(1) Å*; c: 16.4(6)-16.6(6) *Å*; β: 92.1(1)-91.9(1) deg.,
   ii) crystals having the features of i) and having 3 or 4.5 water molecules for each rifaximin molecule,
   iii) crystals having monoclinic space group P2₁ and cell parameters comprised in the ranges:
      a: 14.2(1)-14.5(1) *Å*; b: 19.7(1)-20.1(1) *Å*; c: 16.1(1)-16.2(1) *Å*; β: 108.7(1)-111.4(1) deg.
   iv) crystals having the features of iii) and having zero or 0.5 or 1.5 water molecules for each rifaximin molecule, or
   v) rifaximin α, β, γ, δ.
4. The pharmaceutical composition according to one of items 1 to 3, wherein rifaximin is in a dosage from 20 mg to 1200 mg.
5. The pharmaceutical composition according to one of items 1 to 4, wherein the amino acid(s) is/are selected from aliphatic amino acids, aromatic amino acids, basic amino acids, heterocyclic amino acids, branched amino acids, cyclic amino acids, acid amino acids, amide amino acids or mixtures thereof.
6. The pharmaceutical composition according to one of items 1 to 4, wherein the composition comprises at least one aromatic or heterocyclic amino acid.
7. The pharmaceutical composition according to one of items 1 to 5, wherein the amino acid(s) is/are selected from serine, histidine, tryptophan, valine, leucine or isoleucine.
8. The pharmaceutical composition according to one of items 1 to 4, wherein the amino acid(s) is/are selected from branched chain amino acids.
9. The pharmaceutical composition according to one of items 1 to 3, comprising rifaximin and valine, leucine or isoleucine in a molar ratio of 10:1 with respect to rifaximin.
10. The pharmaceutical composition according to one of items 1 to 9, wherein the pharmaceutically acceptable ingredients include diluting agents, binding agents, disintegrating agents, lubricating agents, release-controlling polymers or bioadhesive polymers.
11. The pharmaceutical composition according to one of items 1 to 10, wherein the composition is in the form of a tablet, capsule, cream, suspension, solution, granulates, ointment or suppository suitable for human or animal administration.
12. The pharmaceutical composition according to one of items 1 to 11, wherein the composition is in a form for oral administration.
13. A pharmaceutical composition according to one of items 1 to 12 for use in the treatment or prevention of bowel infections, diarrhoea, irritable bowel syndrome, bacterial growth in small intestine, Crohn's disease, hepatic insufficiency, hepatic encephalopathy, enteritis, fibromyalgia.
14. A pharmaceutical composition according to one of items 1 to 4, wherein at least one amino acid is a branched chain amino acid for use in the treatment of hepatic encephalopathy.
15. A process for the preparation of a pharmaceutical composition according to one of items1 to 12 comprising the steps:
   - mixing rifaximin and amino acids;
   - adding the excipients and mixing the final mixture in a V mixer for a time between 10 and 30 minutes and
   - granulating in a roller compactor.
16. The process according to item 15 wherein rifaximin and amino acid are mixed and then added to excipients for the obtainment of the composition as tablets, granulates, ointments, creams, suppositories, solution.
17. Use of an amino acid for obtaining a pharmaceutical composition having a rifaximin concentration from 4.5 µg/ml to 60 µg/ml.
18. Rifaximin crystals characterized in that they are obtained by means of a process comprising:
   a) dissolution of the compositions of rifaximin and amino acids, wherein amino acids and rifaximin are in a molar ratio comprised between 1:1 and 10:1 according to item 1, in solutions formed by ethanol/water, in a volumetric ratio comprised between 1:1 and 1:10 (v/v);
   b) evaporation of the solution obtained in step a) at temperatures comprised between room temperature and 40°C, in a time period comprised between 1 and 10 days;
   wherein the resulting crystals have monoclinic space group *P*2₁ and cell parameters comprised in the ranges:
   a: 13.7(1)-13.8(1) *Å*; b: 19.7(1)-19.9 *(1) Å*; c: 16.4(6)-16.6(6) *Å*; β: 92.1(1)-91.9(1) deg.
19. Rifaximin crystals according to item 18, characterized in that they have
   i) 3 water molecules for each rifaximin molecule, or
   ii) 4.5 water molecules for each rifaximin molecule.
20. Rifaximin crystals characterized in that they are obtained by means of a dehydration process of the crystals obtained according to item 18, in the presence of dehydrating agents, wherein the resulting crystals have monoclinic space group *P*2₁ and cell parameters comprised in the ranges:
   a: 14.2(1)-14.5(1) *Å* ; b: 19.7(1)-20.1(1) *Å*; c: 16.1(1)-16.2(1) *Å*; β: 108.7(1)-111.4(1) deg.
21. Rifaximin crystals according to item 20, characterized in that they have
   i) zero water molecules for each rifaximin molecule, or
   ii) 0.5 water molecules for each rifaximin molecule, or
   iii) 1.5 water molecules for each rifaximin molecule.
22. Process for the production of rifaximin crystals, characterized in that it comprises:
   a) dissolution of the compositions of rifaximin and amino acids, wherein amino acids and rifaximin are in a molar ratio comprised between 1:1 and 10:1 according to item 1, in solutions formed by ethanol/water, in a volumetric ratio comprised between 1:1 and 1:10 (v/v);
   b) evaporation of the solution obtained in step a) at temperatures comprised between room temperature and 40°C, in a time period comprised between 1 and 10 days;
   wherein the resulting crystals are characterized by monoclinic space group *P*2₁ and cell parameters comprised in the ranges:
   a: 13.7(1)-13.8(1) *Å*; b: 19.7(1)-19.9 *(1) Å*; c: 16.4(6)-16.6(6) *Å*; β: 92.1(1)-91.9(1) deg.
23. Process for the production of rifaximin crystals, characterized in that it comprises:
   a) dissolution of the compositions of rifaximin and amino acids, wherein amino acids and rifaximin are in a molar ratio comprised between 1:1 and 10:1 according to item 1, in solutions formed by ethanol/water, in a volumetric ratio comprised between 1:1 and 1:10 (v/v);
   b) evaporation of the solution obtained in step a) at temperatures comprised between room temperature and 40°C, in a time period comprised between 1 and 10 days, in the presence of dehydrating agents;
   wherein the resulting crystals have monoclinic space group *P*2₁ and cell parameters comprised in the ranges:
   a: 14.2(1)-14.5(1) *Å* ; b: 19.7(1)-20.1(1) *Å*; c: 16.1(1)-16.2(1) *Å*; β: 108.7(1)-111.4(1) deg.
24. Use of amino acids and rifaximin in a molar ratio comprised between 1:1 and 10:1 in order to obtain rifaximin crystals, characterized by monoclinic space group *P*2₁ and cell parameters comprised in the ranges:
   a: 13.7(1)-13.8(1) *Å*; b: 19.7(1)-19.9 *(1) Å*; c: 16.4(6)-16.6(6) *Å*; β: 92.1(1)-91.9(1) deg.
25. Use of amino acids and rifaximin in a molar ratio comprised between 1:1 and 10:1 in order to obtain rifaximin crystals, characterized by monoclinic space group *P*2₁ and cell parameters comprised in the ranges:
   a: 14.2(1)-14.5(1) *Å* ; b: 19.7(1)-20.1(1) *Å*; c: 16.1(1)-16.2(1) *Å*; β: 108.7(1)-111.4(1) deg.

### Detailed description of the invention

The object of the present invention is represented by rifaximin compositions comprising amino acids and rifaximin, wherein amino acids and rifaximin are in a molar ratio comprised between 1:1 and 10:1, preferably between 1:1 and 5:1, respectively.

The compositions can be in solid form or in aqueous solutions.

Pharmaceutical compositions comprising rifaximin and amino acids, wherein the amino acids are in a molar ratio to rifaximin comprised between 1:1 and 10:1, preferably comprised between 1:1 and 5:1 and provide increased rifaximin solubility from 1.5 to 20 times, in aqueous solutions at room temperature, and from 1.1 to 10 times at 37°C according to the chosen amino acid, in respect to the solution without any amino acid.

The increase of rifaximin solubility in the presence of amino acids is observed when the compositions comprise rifaximin in form of raw rifaximin, amorphous rifaximin, rifaximin polymorph or their mixtures.

When amino acids are in solution with low volumetric percentages of organic solvents, in particular from 1% to 25% (v/v), a synergistic effect of amino acids and organic solvents on the increasing of rifaximin solubility is observed and the solubility increases up to thousand time reaching concentrations higher than 30 mg/ml in solution, in respect to the organic solution without amino acids.

The property of the synergistic effect of the amino acids and organic solvents in the increasing the aqueous rifaximin solubility it is also an advantage for the manufacturing process of the pharmaceutical compositions, because organic solvent can be avoided or reduced. The synergistic effect given by amino acids in the presence of small volume of organic solvent allows the obtainment of solution with higher concentration of rifaximin, in respect to the separate solutions and the solution with high rifaximin concentration allow the conglomerate obtainment.

Amino acids comprised in rifaximin compositions of the present invention, can be aliphatic as glycine, alanine, branched as valine, leucine, isoleucine; hydroxyl or sulfur containing amino acid as cysteine, threonine, methionine; cyclic as proline; aromatic phenylalanine, tyrosine and tryptophan; basic as histidine, lysine, arginine; acid as aspartic acid and glutammic acid and amide as asparagine and glutamine.

The use of different amino acids leads to different available rifaximin concentrations, and it is possible to modulate the rifaximin solubility for different type of disease.

It has been found that aromatic amino acids or amino acid comprising a heterocyclic ring, provide higher solubility of rifaximin.

In particular it has been found that tryptophan and histidine exert a higher solubilization of rifaximin.

Compositions of the present invention comprise rifaximin in form of hydrate, solvate, polymorphous, amorphous or their mixture and amino acids, wherein amino acids and rifaximin are in a molar ratio preferably comprised between 1:1 and 10:1, more preferably between 1:1 and 5:1, can be used together with pharmaceutically acceptable excipients for the preparation of pharmaceutical compositions in solid or liquid form.

In humans and animals, amino acids have important roles as metabolic intermediates and when they are taken into human body from the diet, the amino acids either are used to synthesize proteins or other biomolecules are they are oxidized to urea and carbon dioxide as a source of energy.

Amino acids also have an energetic and nourishing effect, therefore the use of amino acids in pharmaceutical compositions comprising rifaximin for the treatment of all pathologies with related debilitative diseases leads to a beneficial effect for the patient.

Compositions comprising mixtures of amino acids are described in the present invention and in particular compositions comprising aromatic amino acid and branched amino acids such as leucine, isoleucine and valine are useful to increase solubility of rifaximin and beneficial in the treatment of hepatic encephalopathy. These compositions have the advantage to make rifaximin available at higher concentration in respect to the prior art and to be advantageous in particular in the treatment of hepatic encephalopathy.

The compositions of rifaximin and amino acids, object of the present invention, also have the advantage of providing for a larger amount of soluble rifaximin and therefore a higher concentration of rifaximin available in the site of action, thus allowing a better efficacy and/or the total amount of drug administered. In particular, the addition of amino acids to rifaximin in gastroresistant and/or controlled release compositions allows the release of higher rifaximin concentrations in the intestinal tract wherein the infection is localized.

Compositions of the present invention can also comprise rifaximin and amino acids in form of conglomerate of rifaximin and amino acids, wherein the term "conglomerate" means the solid material obtained by drying an aqueous solution of rifaximin and amino acid.

The amino acids and rifaximin conglomerates to be comprised in the pharmaceutical compositions can be obtained by drying under evaporation, aqueous solutions in a ratio from 1:1 to 10:1 with respect of rifaximin. These conglomerates can be added to the pharmaceutical excipients for the preparation of the desired form and they also provide single rifaximin crystals, characterized to be in the form of pure crystal.

The rifaximin crystals, so obtained, are useful as analytical standards.

The amino acids-rifaximin compositions can also comprise conglomerates of rifaximin with acceptable pharmaceutical excipients and their preparations comprise the step of the obtainment of conglomerate and the step of the preparation of pharmaceutical compositions comprising conglomerate with pharmaceutically acceptable excipients.

Rifaximin and amino acid or conglomerate of rifaximin and amino acids, can be comprised in granules optionally coated with controlled release agents and the granules together with extragranular excipients can be used for pharmaceutical preparations. The term "acceptable ingredients" includes pharmaceutically acceptable material, compositions or vehicle, such as a liquid or solid filler, diluent, excipients, solvent or encapsulating material involved in the human or animal use.

Diluting agents, disintegrating agents, lubricating agents, polymers for conferring gastroresistance or controlled release are comprised among the excipients useful in the preparation of pharmaceutical compositions.

The diluting agents useful in the preparation of pharmaceutical compositions are chosen in the group comprising cellulose, microcrystalline cellulose, calcium phosphate, starch, kaolin, dehydrated calcium sulphate, calcium carbonate, lactose, saccharose, glucose, sorbitol, mannitol.

The binding agents useful in the preparation of pharmaceutical compositions are chosen in the group comprising cellulose, cellulose derivatives, carboxymethyl cellulose, microcrystalline cellulose, hydroxypropyl cellulose, hydroxyethyl cellulose, hydroxypropyl methylcellulose, starches, potato starch, maize starch, partially gelatinized starch, gums, synthetic gum, natural gums, polyvinylpyrrolidone, polyethylene glycol, gelatin, polyols, propylene glycol, alginates, sugars or their mixtures.

The disintegrating agents useful in the preparation of pharmaceutical compositions are chosen in the group comprising sodium carboxymethyl cellulose, also called carmelose sodium, cross-linked sodium carboxymethyl cellulose, also called croscarmelose sodium, polyvinylpyrrolidone, also called povidone, cross-linked polyvinylpyrrolidone, also called crospovidone, starch, pregelatinized starch, silica and lubricating agents, chosen among magnesium or calcium stearate, sodium stearyl fumarate, hydrogenated vegetable oils, mineral oils, polyethylene glycols, sodium lauryl sulphate, glycerides, sodium benzoate.

The lubricating agents useful in the preparation of pharmaceutical compositions are chosen in the group comprising silica, magnesium or calcium stearate, sodium stearyl fumarate, hydrogenated vegetable oils, mineral oils, polyethylene glycols, sodium lauryl sulphate, glycerides, glyceryl dibehenate, glycerol stearate.

The polymers suitable for obtaining a controlled release can have a synthetic or natural origin. The polymers suitable for the preparation of the pharmaceutical compositions are chosen in the group comprising copolymers of acrylic acid, such as the copolymer methacrylic acid-ethyl acrylate 1:1, copolymer of methacrylic acid with an acrylic or methacrylic ester such as the copolymer methacrylic acid-ethyl acrylate 1:1 and the copolymer methacrylic acid-methyl methacrylate 1:2, polyvinyl acetate phtalate, hydroxy propyl methyl cellulose phtalate and cellulose acetate phtalate, commercially available products, for instance with the trademarks Kollicoat^{®}, Eudragit^{®}, Aquateric^{®}, Aqoat^{®}; natural polymers like shellac, commercially available with the trademark Aquagold^{®} (shellac 25%) and ethyl cellulose.

The pharmaceutical compositions can also have bioadhesive or mucoadhesive properties in order to adhere to intestinal mucosa.

Examples of polymers, oligomers or their mixtures which can confer bioadhesive properties are chosen in the group comprising: pectins, zeins, casein, gelatin, albumin, collagen, kitosan, oligosaccharides and polysaccharides such as, for instance, cellulose, dextran, polysaccharides from tamarind seeds, xanthan gum, arabic gum, hyaluronic acid, alginic acid, sodium alginate.

When the bioadhesive polymer is a synthetic polymer, the polymer is chosen among polyamides, polycarbonates, polyalkylenes, polyalkylene glycols, polyalkylene oxides, polyalkylene terephthalates, polyvinyl alcohols, polyvinyl ethers, polyvinyl esters, polyvinylpyrrolidone, polysiloxanes, polyurethanes, polystyrenes, polymers of acrylic acid and methacrylic esters, copolymer of methacrylic acid-ethyl acrylate, polylactides, barbituric polyacids, polyanhydrides, polyorthoesters and their mixtures.

Further useful polymers are methylcellulose, ethylcellulose, hydroxypropyl cellulose, hydroxybutyl methylcellulose, cellulose acetate, cellulose propionate, cellulose acetate butyrate, cellulose acetate phthalate, carboxy methyl cellulose, cellulose triacetate, cellulose sulfate sodium salt, polymethyl methacrylate, poly isobutyl acrylate, poly octadecyl acrylate, polypropylene, polyethylene glycol, polyethylene oxide, polyethylene terephthalate, polyvinyl acetate, polyvinyl chloride, polystyrene, polyvinylpyrrolidone, polyvinyl phenol and their mixtures.

Another group of polymers useful in the obtainment of bioadhesivity are polymers having a branch with at least one bonded hydrophobic group, wherein hydrophobic groups generally are non-polar groups. Examples of said hydrophobic groups comprise alkyls, alkenyls and alkyl groups. Preferably, hydrophobic groups are chosen to increase polymers bioadhesivity. Other polymers are characterized by hydrophobic branches with at least one hydrophilic group, such as carboxylic acids, sulphonic acids and phosphonic acids, neutral and positively charged amines, amides and imines, wherein the hydrophilic groups are such to increase the polymer bioadhesivity.

Pharmaceutical compositions comprising rifaximin and amino acids can optionally comprise also edulcorating agents, coloring agents, anti-oxidizing agents, buffering agents and flavoring agents.

Edulcorating/sweetening agents useful in the preparation of pharmaceutical compositions are chosen in the group comprising potassium acesulfame, sorbitol, mannitol, isomalt, maltitol, lactitol, xylitol, aspartame, cyclamic acid, cyclamate salts, lactose, sucralose, saccharine and saccharine salts.

When the amino acids- rifaximin compositions are administered as pharmaceuticals, to humans and animals, they can be given per se or as a pharmaceutical composition containing, for example, and they can comprise from 0.1 to 90% of active ingredient in combination with a pharmaceutically acceptable carrier.

The pharmaceutical compositions comprising rifaximin and amino acids together with pharmaceutically acceptable excipients can be in the form of granulates, tablets, capsules, creams, ointments, suppository, suspensions or solutions of rifaximin for human and animal administration.

The pharmaceutical compositions comprising rifaximin and amino acids in an amount between 20 and 1200 mg, preferably between 100 and 600 mg, are useful in the prevention and treatment of traveler's diarrhea, hepatic insufficiency, hepatic encephalopathy, infectious diarrhea, diverticular disease, an antibacterial prophylactic prior and post colon surgery, irritable bowel syndrome, Crohn's disease, *Clostridium difficile-*associated diarrhea, small intestinal overgrowth, traveler's diarrhea prophylaxis, dysentery, pauchitis, peptic ulcer disease, surgical prophylaxis, gastric dyspepsia enteritis, fibromyalgia and vaginal infections.

Actual dosage levels and time course of administration of the active ingredients in the pharmaceutical compositions may be varied so as to obtain an amount of the active ingredient which is effective to achieve the desired therapeutic response for a particular subject, composition, and mode of administration, without being toxic to the subject. An exemplary dose range is from 20 to 3000 mg per day. Other doses include, for example, 600 mg/day, 1100 mg/day and 1650 mg/day. Other exemplary doses include, for example, 1000 mg/day, 1500 mg/day, from between 500 mg to about 1800 mg/day or any value in-between.

The amino acids dosage is in a molar ratio between 1 to 10 in respect to the rifaximin, preferably between 1 to 5 and when branched amino acids aminoacid are comprised in the composition they are in a molar ratio between 1 to 10 in respect to the rifaximin.

The pharmaceutical compositions of the invention can be formed by various method in the art such as granulation, direct compression, double compression. In a preferred embodiment the processes for making the pharmaceutical composition of the invention, wherein amino acids are in a molar ratio between 10:1 and 5:1, in respect to the rifaximin, comprise the step of mixing rifaximin and amino acids for obtaining an homogeneous mixture and the addition of excipients for the preparation in solid or liquid form for oral administration, for example tablets, powder, granules, pastes, capsules; parenteral administration, for example, by subcutaneous, intramuscular or intravenous injections, for example, a sterile solution or suspension; topical application, for example, as a cream, ointment or spray applied to the skin; intravaginally or intrarectally, for example, as a pessary, cream or a foam; or aerosol.

Conglomerates of rifaximin and amino acids allow the storage of rifaximin without any rifaximin transformation and also they are useful to obtain single crystals.

The preparation of rifaximin crystals in the conglomerates comprises the first step of mixture of rifaximin and amino acids in aqueous solutions, the second step of complete evaporation of solvent at room temperature or at temperatures comprised between room temperature and 100°C, and the final step of drying in the presence or not of dehydrating agents at ambient pressure or under vacuum. The analyzed crystals are characterized by having high purity and their sizes are suitable for structure characterization via single crystal X-ray diffraction. The crystals are parallelepiped or cube and their sizes are comprised from 0.1 to 0.3 mm x 0.1 to 0.3 mm x 0.1 to 0.3 mm.

The compositions of rifaximin and amino acids allow to obtain rifaximin crystals. These latter are obtained when the compositions of rifaximin and amino acids, wherein amino acids and rifaximin are in molar ratio comprised between 1:1 and 10:1, preferably between 1:1 and 5:1, are dissolved in aqueous solutions comprising alcohols in a volume ratio water/alcohol comprised between 1:1 and 10:1 (v/v), preferably 5:1 (v/v), wherein the rifaximin concentration in solution is higher than 15 mg/ml.

Rifaximin crystals are obtained by means of the process comprising a low evaporation of the aforesaid solutions of rifaximin and amino acids, at room temperature or at temperatures comprised between room temperature and 100°C, and then their drying, possibly in the presence of dehydrating agents. The analyzed crystals are characterized in that they have high purity and sufficient size for being analyzed by means of single crystal X-ray diffraction.

Said crystals are obtained when the rifaximin comprised in the compositions is raw rifaximin, amorphous rifaximin, pure rifaximin polymorphs or their mixtures in the presence of single amino acids or their mixtures.

Rifaximin crystals have been characterized by means of the X-ray diffraction technique, which allowed to obtain cell parameters and structural details (atomic coordinates, connectivity, distances and bond angles).

So obtained rifaximin crystals, analyzed by means of X-ray diffraction, are characterized in that they have cell parameters a, b, c, and α, β and γ comprised in the ranges reported in Table 1.

**Table 1**

| **Values of cell parameters** | |
|---|---|
| a/Å | 13.7(1) - 13.8(1) |
| b/A | 19.7(1) - 19.9(1) |
| c/Å | 16.4(6) - 16.6(6) |
| α/deg | 90 |
| β/deg | 92.1(1) - 91.9(1) |
| γ/deg | 90 |

In particular, rifaximin crystals have been found which are characterized by the following cell parameters:
- crystal 1: a: 13.7960(8) A; b: 19.944 (4) A; c: 16.607(6) A; β: 92.180(1) deg; α and γ: 90 deg;
- crystal 2: a: 13.753(8) A; b: 19.749 (4) A ; c: 16.378(6) A; β: 91.972(1) deg; α and γ: 90 deg.

Knowing cell parameters and structural details it is possible to calculate the theoretical diffractogram which is then compared to the experimental one obtained from the powders. This comparison shows that the crystals described in Table 1, and crystals 1 and 2 are crystals of rifaximin β. Crystal 1 contains only, and stechiometrically, 3 water molecules for each rifaximin molecule, and is called rifaximin β_{3.0} (where the subscript indicates the number of water molecules for each rifaximin molecule); crystal 2 contains only, and stechiometrically, 4.5 water molecules for each rifaximin molecule, and is called rifaximin β_{4.5}.

Other rifaximin crystals have been obtained by means of controlled drying of solutions comprising rifaximin and amino acids, or by means of direct drying (P₂O₅) of crystals of rifaximin β, wherein the size of these crystals is large enough to be analyzed by single crystal X-ray diffraction.

So obtained rifaximin crystals, analyzed by means of X-ray diffraction, are characterized in that they have cell parameters a, b c and α, β and γ comprised in the ranges reported in Table 2.

**Table 2**

| **Values of cell parameters** | |
|---|---|
| a/Å | 14.2(1) - 14.5(1) |
| b/A | 19.7(1) - 20.1(1) |
| c/Å | 16.1(1) - 16.2(1) |
| α/deg | 90 |
| β/deg | 108.7(1) - 111.4(1) |
| γ/deg | 90 |

In particular, rifaximin crystals have been found which are characterized by the following cell parameters:
- crystal 3: a: 14.232(4) A; b: 19.822 (4) A; c: 16.164(4) A; β: 108.74(3) deg; α and γ: 90 deg;
- crystal 4: a: 14.579(4) A; b: 20.232 (4) A; c: 16.329(4) A; β: 111.21(3) deg; α and γ: 90 deg;
- crystal 5: a: 14.492(4) A; b: 20.098 (4) A; c: 16.215(4) A; β: 111.21(3); α and γ: 90 deg.

Knowing cell parameters and structural details it is possible to calculate the theoretical diffractogram which is then compared to the experimental one obtained from the powders. This comparison shows that the crystals previously described in Table 2, crystal 3, crystal 4 and crystal 5 are crystals of rifaximin α.

In particular, crystal 3 does not contain any water molecule, and is called α₀; crystal 4 contains, only and stechiometrically, 0.5 water molecules for each rifaximin molecule and is called rifaximin α_{0.5}; crystal 5 contains, only and stechiometrically, 1.5 water molecules for each rifaximin molecule and is called rifaximin α_{1.5}.

Rifaximin single crystals are obtained when the rifaximin and amino acid are mixed in aqueous solution; the rifaximin can be raw rifaximin, amorphous rifaximin, rifaximin polymorph or their mixtures.

Rifaximin crystals have been characterized by means of the X-ray diffraction technique, which allows to obtain cell parameters and structural details atomic coordinates, connectivity, bond distances and angles.

On the basis of cell parameters and structural details it is possible to calculate the theoretical diffractogram which is then compared to the experimental one obtained from the powders.

Rifaximin crystals obtained by the rifaximin and amino acid conglomerates are crystals of rifaximin β and in particular they have been obtained a crystal of β form with 3 water molecules for each rifaximin molecule, called rifaximin β_{3.0}; a crystal of β form with 4.5 water molecules for each rifaximin molecule, called rifaximin β_{4.5}.

Other rifaximin crystals have been obtained by means of controlled drying of solutions comprising rifaximin and amino acids, or by means of direct drying (P₂O₅) of crystals of rifaximin β, wherein the size of these crystals is large enough to be analyzed by single crystal X-ray diffraction.

On the basis of cell parameters and structural details it is possible to calculate the theoretical diffractogram which is then compared to the experimental one obtained from the powders.

The obtained crystal are rifaximin α and in particular it has been obtained a crystal which does not contain any water molecule, called α₀; a crystal with 0.5 water molecules for each rifaximin molecule, called rifaximin α_{0.5} and a crystal with 1.5 water molecules for each rifaximin molecule, called rifaximin α_{1.5}.

The crystalline structures of rifaximin β_{4.5}, β₃, α_{1.5} and α_{0.5} have the characteristic of containing at least one water molecule for each dimeric unit, characterized in that it interacts by means of a hydrogen bond with the amide nitrogen at position 14; this water molecule enters the *ansa* chain of the rifaximin structure.

In the microcrystalline powders described in the literature, the diffraction profile is given by the superimposition of the diffraction profiles of single crystallites constituting the powder, each of them being characterized by a possible different content of crystallization water. Therefore, the relevance of having obtained rifaximin crystals with a suitable size for structural analysis by means of single crystal X-ray diffraction is given by the fact that, knowing the structural parameters, it is possible to calculate rifaximin diffractograms corresponding to different contents of crystallization water.

Single rifaximin crystals can therefore be used as analytical standards in crystallographic analysis for the quantitative and qualitative determination of rifaximin mixtures, even complex ones, wherein crystals characterized by a different water content and by cell parameters within the ranges reported in Tables 1 and 2 are present in variable proportion.

The number and position of water molecules within rifaximin crystals can have an influence on the parameters of the unit cell and on the position of the peaks in the X-ray diffractogram. The obtained crystals allow to determine the presence of a polymorphic form, even in complex mixtures.

The parameters characterizing the rifaximin crystals described in Examples 2, 4, 6 and 8 have been obtained in laboratory by means of an Oxford Diffraction X'calibur diffractometer with MoKα radiation (λ=0.71073 Å) or by means of an XRD1 line at the Elettra Synchrotron in Trieste.

Rifaximin single crystals are useful in quantitatively and qualitatively determining the presence of such polymorphic forms in complex rifaximin mixtures, in production batches and in finished pharmaceutical compositions comprising rifaximin, and can also be useful in determining the amorphous amount in a powder mixture.

The use of amino acid is also advantageous in industrial process for the preparation of rifaximin pharmaceutical compositions, the increasing of solubility in aqueous solution avoid or reduce the volume of organic solvents to be used. The avoiding or reduction of organic solvents is very important because when the product is addressed to human or animals the Health Authorities require severe controls for the residual organic solvents.

Moreover the reduction of organic solvent makes safer the industrial processes, because less quantities of organic solvents reduce flash point of the solution.

Another relevant aspect of the present invention is represented by the use of amino acids for obtaining compositions which allow the obtainment of rifaximin crystals and for increasing the rifaximin solubility in solutions containing water among solvents. The rifaximin compositions in the presence of amino acids, wherein the amino acids are pure or in mixtures, in a molar ratio to rifaximin comprised between 1:1 and 10:1, preferably comprised between 1:1 and 5:1, increase from 1.5 to 20 times rifaximin solubility in aqueous solutions at room temperature, and from 1.1 to 10 times at 37°C according to the chosen amino acid.

The increase of rifaximin solubility in the presence of amino acids is observed with raw rifaximin, amorphous rifaximin, rifaximin pure polymorphs or their mixtures in the presence of single amino acids or their mixtures.

It has also been observed that amino acids have a synergic effect on rifaximin solubility in aqueous solutions, in the presence of low percentages of organic solvents, in particular when organic solvents have percentages which are lower or equal to 20% (v/v).

As shown in Example 11, rifaximin in an ethanol/water solution 1:4 (v/v) has a solubility of 48 µg/ml, when amino acids are added to this solution in a molar ratio to rifaximin comprised between 1:3 and 1:5, as shown in Examples 2, 4, 6 and 8 and 10, rifaximin is solubilized at concentrations which are nearly thousand times higher, thus reaching concentrations higher than 30 mg/ml in solution.

The possible use of water/ethanol solutions with a low ethanol content in the production process of crystalline rifaximin represents an advantage from the point of view of the process safety. In fact, said solutions have a higher point of inflammability, also called "flash point", defined as the minimum temperature at which, at room pressure, a liquid produces vapors in such an amount that, together with air, they form a mixture which can flare up or explode. Therefore, the higher the flash point, the safer the process.

In the case of 20% ethanol solutions (v/v) in water, as in Example 2, the flash point is 36°C, whereas for 70% ethanol solutions, like those described in the known art for rifaximin crystallization, the flash point decreases to 21 °C.

Moreover, another advantage is represented by the fact that in these solutions having a low ethanol content, rifaximin crystallization can be coupled to amino acids crystallization, thus obtaining both crystallizations and their mixture in the solid state in a single step.

Different amino acids lead to different available rifaximin concentrations, thus allowing the modulation of rifaximin solubility.

Examples of the present invention describe the preparation of solid compositions comprising different amino acids in a different molar ratio with respect to the rifaximin, in form of granules for ready suspension and/or tables. In particular are described preparation of solid compositions comprising rifaximin and tryptophan, serine and histidine in a molar ratios comprised between 10 and 1 with respect to the rifaximin.

Are described compositions in form of granules for ready suspension or for tablets preparations and said granules can include or coated with agents for controlling release.

The composition can be also in form of tablets prepared by mixing the components and they can be coated with coating film or film to provide controlled release.

Examples of the invention also demonstrate the increase of rifaximin solubility when amino acids are comprised in the composition and also a synergic effect of the amino acids in the presence of low volume of organic solvents in the rifaximin solubilization.

The Examples also demonstrate that the effect of amino acids in the increasing of rifaximin solubility is higher than the solubility of rifaximin composition in subject in fastly and feedly conditions.

Example 1 describes the preparation of solid compositions of rifaximin and amino acids wherein different amino acids, such as tryptophan, serine and histidine, are mixed in molar ratios comprised between 1 e 5 if compared to rifaximin in amorphous form or polymorph α.

Example 2 describes the preparation of rifaximin crystals by means of a process comprising the solubilization of composition A of Example 1 in a solution ethanol/water 1:4 (v/v), wherein rifaximin reaches a concentration in solution corresponding to 40 mg/ml. A solid mass, which can be defined as a conglomerate, an assembly of distinguishable and separable crystals of rifaximin and amino acids, is obtained by slow evaporation of the solution.

Example 3 describes the structural characterization, by means of X-ray diffraction from a conventional source or from a synchrotron, of rifaximin crystals obtained from Example 2. The structural resolution allows to establish that the analyzed crystals are crystals of rifaximin β, characterized in that they have 3 and 4.5 water molecules.

Examples 4 and 5 describe the preparation and characterization of rifaximin crystals obtained by solubilization of the composition B of Example 1 in a solution ethanol/water 1:4 (v/v), wherein rifaximin reaches a concentration corresponding to 40 mg/ml.

Examples 6 and 7 describe the preparation and characterization of rifaximin crystals obtained from the composition C of Example 1.

Examples 8 and 9 describe the preparation and characterization of rifaximin crystals obtained from composition D of Example 1.

Example 10 describes the preparation of crystals of rifaximin α obtained by transforming the rifaximin crystal obtained in Example 2 in the presence of dehydrating agents. The same result can be obtained by drying the solid mass comprising crystals of rifaximin and amino acids obtained according to Examples 2, 4, 6, and 8 in the presence of dehydrating agents or under vacuum and at temperatures comprised between room temperature and 40°C.

Comparative Example 11 demonstrates that, in the absence of amino acids, rifaximin in a solution ethanol/water 1:4, reaches a maximum concentration of 48 µg/ml.

Example 12 shows a comparison of rifaximin solubility in aqueous solutions with or without amino acids at various temperatures. Rifaximin solubility in aqueous solutions turns out to be about 3 µg/ml at room temperature and about 7 µg/ml at 37°C, whereas in the presence of amino acids its solubility is higher than 30 µg/m in both cases. This result allows the preparation of pharmaceutical compositions wherein rifaximin is more available.

Example 13 describes the solubility of the solid mass, defined as a conglomerate of amino acids and crystals of rifaximin, prepared according to Example 2, which in buffer solution at pH 6.8 reaches a rifaximin concentration corresponding to about 30 µg/ml.

These examples demonstrate that the compositions object of the present invention lead to a suitable concentration of rifaximin in water if compared to the examples wherein amino acids are not present.

Examples 14 and 15 have been carried out as a comparison. These examples determine rifaximin solubility in water and buffers at room temperature and at 37°C, in the absence of organic solvents and amino acids.

Example 14 describes rifaximin solubility in water and in buffers at pH 4, pH 7 and pH 10, thus demonstrating that rifaximin is substantially insoluble in water, in particular at those pH values which are similar to the physiologic ones.

Example 15 describes rifaximin solubility obtained by means of the dissolution test of coated tablets and tablets comprising rifaximin gastroresistant granules, in solutions simulating intestinal fluids before meals, FaSSIF solutions, and after meals, FeSSIF solutions.

Coated rifaximin tablets (Normix^{®}) show rifaximin solubility values in FaSSIF solutions of about 8 µg/ml after 360 minutes, whereas in FeSSIF solutions rifaximin concentration is about 11 µg/ml after 360 minutes.

The tablets comprising gastroresistant granules in rifaximin show a rifaximin solubility in FaSSIF solutions of about 13 µg/ml after 360 minutes, whereas in FeSSIF solutions rifaximin concentration is about 20 µg/ml after 360 minutes.

The examples of the invention show that the effect exerted by amino acids on increasing rifaximin solubility exceeds the one provided by intestinal fluids, therefore the addition of amino acids to gastroresistant and non gastroresistant compositions allows to obtain higher rifaximin concentrations, in particular after meals.

### Example 1

### Preparation of solid compositions comprising rifaximin and amino acids

A rifaximin amount corresponding to 200 mg was mixed in a V-mixer together with respective amounts of amino acid (AA) as reported in Table 3.

**Table 3**

| **Composition** | **Amino acid (AA)** | **Rifaximin form** | **Molar ratio AA:rifaximin** |
|---|---|---|---|
| A | Tryptophan | Rifaximin α | 4:1 |
| B | Serine | Rifaximin α | 3:1 |
| C | Histidine | Rifaximin α | 4:1 |
| D | Histidine | Amorphous Rifaximin | 4:1 |

The obtained mixture can be kept at room temperature without any particular further precaution beside those taken for keeping rifaximin or amino acids alone.

### Example 2

### Preparation of crystals of rifaximin β starting from the composition A of Example 1

5 ml of a solution formed by ethanol/water in volumetric ratio 1:4 (v/v) were added to composition A of Example 1; the solution was then heated at 100°C until reaching complete dissolution, and left for complete solvent evaporation at room temperature for 4 days. It was observed the formation of rifaximin conglomerates characterized by the contemporary presence of rifaximin crystals and tryptophan crystals.

### Example 3

### Analysis of crystals of rifaximin β_{3.0} and β_{4.5} obtained in Example 2

Rifaximin crystals obtained in Example 2 were separated from amino acids and measured by X-ray diffraction using:
a) An X'calibur diffractometer by Oxford Diffraction, provided with a CCD area detector which uses MoKα radiation (λ = 0.71073 A) and a graphite monochromator; data were collected at room temperature. The structures were solved by direct methods using the SHELX97 program (Sheldrick, 2008) implemented in the WinGX package (Farrugia, 1999);
b) synchrotron ELETTRA (Trieste) at the XRD1 beam line at room temperature and at 295 K, using the cooling system MARSCH 300.

The structures were solved using the SHELX97 program (Sheldrick, 2008) implemented in the WinGX package (Farrugia, 1999). Table 4 reports the crystallographic parameters relating to the analyzed rifaximin crystals.

**Table 4**

| | **Crystallographic parameters** | **Crystallographic parameters** |
|---|---|---|
| Chemical formula | C₄₃H₅₇N₃O₁₄ | C₄₃H₆₀N₃O_{15.5} |
| H₂O molecules for each rifaximin molecule | 3.0 | 4.5 |
| MW | 839.93 | 866.95 |
| Temperature/K | 295 | 295 |
| λ(Å) | 0.71073 | 1 |
| Crystalline system | monoclinic | monoclinic |
| Space group | *P*2₁ | *P*2₁ |
| a/Å | 13.7960(8) | 13.753(8) |
| b/A | 19.944(4) | 19.749(4) |
| c/Å | 16.607(6) | 16.378(6) |
| β/deg | 92.180(1) | 91.972(1) |
| V/Å³ | 4566.1 | 4445.8(6) |
| Z | 4 | 4 |
| D_{c}/Mg m⁻³ | 1.222 | 1.295 |

Knowing cell parameters and structural details it is possible to determine that the crystals are crystals of rifaximin β, called rifaximin β_{3.0} and rifaximin β_{4.5}.

### Example 4

### Preparation of rifaximin crystals starting from the composition B of Example 1

5 ml of a solution formed by ethanol/water in a volumetric ratio 1:4 (v/v) were added to composition B of Example 1. The solution was then heated at 100°C until reaching complete dissolution, and left for complete solvent evaporation at room temperature for 4 days. It was observed the formation of rifaximin crystals and serine crystals.

### Example 5

### Analysis of rifaximin crystals obtained in Example 4

Rifaximin crystals obtained in Example 4 were separated from amino acids crystals, and then, for one of them, cell parameters were determined at room temperature by means of an X'calibur diffractometer by Oxford Diffraction using the MoKα radiation (λ = 0.71073 A). Table 5 reports crystallographic parameters relating to the analyzed rifaximin crystal.

**Table 5**

| | **Crystallographic parameters** |
|---|---|
| Temperature/K | 295 |
| Morphology | Orange prism |
| Crystalline system | monocline |
| Space group | P2₁ |
| a/Å | 13.86(1) |
| b/A | 19.90(1) |
| c/Å | 16.69(1) |
| β/deg | 91.85(1) |

Knowing the cell parameters it is possible to determine that the crystal is a crystal of rifaximin β.

### Example 6

### Preparation of rifaximin crystals starting from the composition C of Example 1

5 ml of a solution formed by ethanol/water in a volumetric ratio 1:4 (v/v) were added to composition C of Example 1. The solution was then heated at 100°C until reaching complete dissolution, and left for spontaneous evaporation at room temperature for 4 days. It was observed the formation of rifaximin crystals and histidine crystals.

### Example 7

### Analysis of rifaximin crystals obtained in Example 6

Rifaximin crystals obtained in Example 6 were separated from amino acids crystals, and then, for one of them, cell parameters were determined at room temperature by means of an X'calibur diffractometer by Oxford Diffraction using the MoKα radiation (λ = 0.71073 A). Table 6 reports crystallographic parameters relating to the analyzed rifaximin crystal.

**Table 6**

| | **Crystallographic parameters** |
|---|---|
| Temperature/K | 295 |
| Morphology | Orange prism |
| Crystalline system | monocline |
| Space group | P2₁ |
| a/Å | 13.75(1) |
| b/A | 19.76(1) |
| c/Å | 16.35(1) |
| β/deg | 92.09(1) |

Knowing the cell parameters it is possible to determine that the analyzed crystal is a crystal of rifaximin β.

### Example 8

### Preparation of rifaximin crystals starting from the composition D of Example 1

5 ml of a solution formed by ethanol/water in a volumetric ratio 1:4 (v/v) were added to composition D of Example 1. The solution was then heated at 100°C until reaching complete dissolution, and left for spontaneous evaporation at room temperature for 4 days. It was observed the formation of rifaximin crystals and histidine crystals.

### Example 9

### Analysis of rifaximin crystals obtained in Example 8

Rifaximin crystals obtained in Example 8 were separated from amino acids crystals, and then, for one of them, cell parameters were determined at room temperature by means of an X'calibur diffractometer by Oxford Diffraction using the MoKα radiation (λ = 0.71073 A).

Table 7 reports crystallographic parameters relating to the analyzed rifaximin crystal.

**Table 7**

| | **Crystallographic parameters** |
|---|---|
| Temperature/K | 295 |
| Morphology | Orange prism |
| Crystalline system | monocline |
| Space group | P2₁ |
| a/Å | 13.78(1) |
| b/A | 19.74(1) |
| c/Å | 16.38(1) |
| β/deg | 92.12(1) |

Knowing the cell parameters it is possible to determine that the analyzed crystal is a crystal of rifaximin β.

### Example 10

### Preparation of crystals of rifaximin α₀, α_{0.5} and α_{1.5}

Rifaximin crystals obtained in Example 2 were placed in a dryer at room temperature and pressure in the presence of P₂O₅ for 24 hours.

Rifaximin crystals were analyzed by means of X-ray diffraction using:
a) An X'calibur diffractometer by Oxford Diffraction, provided with a CCD area detector which uses MoKα radiation (λ = 0.71073 A) and a graphite monochromator; data were collected at room temperature. The structures were solved by direct methods using the SHELX97 program (Sheldrick, 2008) implemented in the WinGX package (Farrugia, 1999);
b) synchrotron ELETTRA (Trieste) at the XRD1 beam line at room temperature and at 100 K and 295 K, using the cooling system MARSCH 300.

The structures were solved using the SHELX97 program (Sheldrick, 2008) implemented in the WinGX package (Farrugia, 1999).

Table 8 reports the crystallographic parameters relating to the analyzed rifaximin crystals.

These crystals are characterized in that they are crystals of rifaximin α and in that they have precise water molar ratios.

**Table 8**

| | **Crystal parameters 3** | **Crystal parameters 4** | **Crystal parameters 4** | **Crystal parameters 5** |
|---|---|---|---|---|
| Chemical formula | C₄₃H₅₁N₃O₁₁ | Cr₄₃H₅₁N₃O_{11.5} | Cr₄₃H₅₁N₃O_{11.5} | C₄₃H₅₄N₃O_{12.5} |
| H₂O molecules for each rifaximin molecule | 0 | 0.5 | 0.5 | 1.5 |
| MW | 785.87 | 794.89 | 794.89 | 812.83 |
| temperature/K | 295 | 295 | 100 | 295 |
| λ(Å) | 1 | 0.71073 | 1 | 0.71073 |
| Crystalline system | Monoclinic | monoclinic | monoclinic | monoclinic |
| Space group | *P*2₁ | *P*2₁ | *P*2₁ | *P*2₁ |
| a/Å | 14.232(4) | 14.579(4) | 14.401(4) | 14.492(4) |
| b/A | 19.822(4) | 20.232(4) | 19.662(4) | 20.098(4) |
| c/Å | 16.164(4) | 16.329(4) | 16.153(4) | 16.215(4) |
| β/deg | 108.74(3) | 111.21(3) | 111.04(3) | 111.21(3) |
| V/Å³ | 4318.2(5) | 4402.7(5) | 4268.6(5) | 4402.7(5) |
| Z | 4 | 4 | 4 | 4 |
| D_{c}/Mg m⁻³ | 1.209 | 1.237 | 1.237 | 1.226 |

Analogous results were obtained by drying under the same conditions starting from rifaximin crystals obtained in Examples 4, 6 and 8.

### Example 11

### Determination of rifaximin solubility in ethanol-water solutions

A rifaximin amount corresponding to 200 mg is dissolved in 10 ml of a solution formed by ethanol/water 1:4 (v/v), left for two days under stirring at room temperature.

Rifaximin solubility was determined by spectrophotometric method at wavelength 276 nm with a rifaximin calibration curve, and is of 48 µg/ml.

### Example 12

### Determination of rifaximin solubility in aqueous solutions in the presence of amino acids

The example describes the determination of rifaximin solubility in aqueous solutions in the presence and in the absence of amino acids.

In particular, trials are summarized in Table 9.
- Experiment 1 describes the determination of the solubility obtained by placing 20 mg rifaximin in phosphate buffer at pH 6.8 (P.B.) at room temperature for 2 hours;
- Experiments 2, 3 and 4 describe rifaximin solubility in water obtained by placing 200 mg rifaximin and 195 mg tryptophan, serine and histidine, respectively corresponding to molar ratios rifaximin:amino acid 1:4, 1:7.5 and 1:5, and the solutions are kept under stirring at room temperature for 24 hours.
- Experiments 5-14 describe the solubility obtained by placing 20 g rifaximin in 500 ml phosphate buffer at pH 6.8 with various amino acids in molar ratios reported in Table 9, and the solutions are kept under stirring for 24 hours at 37°C in vessel.

Rifaximin solutions are filtered and rifaximin concentrations are determined by means of calibration curve spectrophotometry, at wavelength 364 nm.

Table 9 also reports values relating to the increase of rifaximin concentrations in solutions with different amino acids if compared to respective reference solutions of rifaximin alone in water or phosphate buffer (Experiments 1 and 14).

**Table 9**

| **Experiment** | **AA** | **V (ml)** | **T (°C)** | **Solution** | **AA/Rifax (mol/ mol)** | **Conc. (µg/ml)** | **Conc. trial N./ Conc. trials 1 and 5** |
|---|---|---|---|---|---|---|---|
| 1 | - | 50 | r.t. | P.B. | - | 3,5 | 1 |
| 2 | Tryptophan | 5 | r.t. | H₂O | 4:1 | 35 | 10 |
| 3 | Serine | 5 | r.t. | H₂O | 7.5:1 | 5.7 | 1.6 |
| 4 | Histidine | 5 | r.t. | H₂O | 5:1 | 21.8 | 6.2 |
| 5 | - | 500 | 37°C | H₂O | - | 7 | 1 |
| 6 | Tryptophan | 500 | 37°C | P.B. | 10:1 | 22 | 3.1 |
| 7 | Tryptophan | 500 | 37°C | P.B. | 3:1 | 30 | 4.2 |
| 8 | Tryptophan | 500 | 37°C | P.B. | 1:1 | 21 | 3 |
| 9 | Histidine | 500 | 37°C | P.B. | 10:1 | 16 | 2.3 |
| 10 | Histidine | 500 | 37°C | P.B. | 3:1 | 12 | 1.7 |
| 11 | Histidine | 500 | 37°C | P.B. | 1:1 | 10 | 1.4 |
| 12 | Valine | 500 | 37°C | P.B. | 3:1 | 8 | 1.1 |
| 13 | Leucine | 500 | 37°C | P.B. | 3:1 | 8 | 1.1 |
| 14 | Isoleucine | 500 | 37°C | P.B. | 3:1 | 10 | 1.4 |

### Example 13

### Determination of solubility in water of conglomerates of rifaximin and tryptophan obtained in Example 2

The solubility of conglomerates of rifaximin and tryptophan, obtained in Example 2, was determined by placing 653 mg of this solid in 5 ml phosphate buffer at pH 6.8 at room temperature and the solution was left for 24 under stirring.

Rifaximin concentration in solution was determined by spectrophotometric method at wavelength 364 nm and is of 28 µg/ml.

### Example 14

### Determination of rifaximin solubility in water and in buffer solutions at various pH (Comparative Example)

Rifaximin solubility values was determined by placing the rifaximin amounts respectively reported in Table 10 in a volume of 50 ml, respectively of water, phosphate buffer at pH 4, phosphate buffer at pH 7 and borate buffer at pH 10.

The suspensions stored in a nitrogen atmosphere and thermostated at 30°C are kept under stirring for 24 hours.

The determination of rifaximin concentration in solution was carried out by means of a chromatographic method under the conditions reported in European Pharmacopeia Ed. 7.1, 04/2011, paragraph 2362, pag.3459 and the obtained results were reported in Table 10.

**Table 10**

| **Trial** | **Solution** | **Rifaximin amount (mg)** | **Rifaximin concentration (µg/ml)** |
|---|---|---|---|
| 1 | H₂O | 20 | 3.63 µg/ml |
| 2 | Phosphate buffer pH 4 | 15 | 4.12 µg/ml |
| 3 | Phosphate buffer pH 7 | 20 | 3.22 µg/ml |
| 4 | Borate buffer pH 10 | 65 | 299 µg/ml |

### Example 15

### Determination of rifaximin dissolution profile in coated tablets and tablets comprising gastroresistant microgranules (Comparative Example)

The dissolution profiles for commercially available coated tablets Normix^{®}, comprising 200 mg rifaximin, and tablets comprising 400 mg rifaximin in gastroresistant microgranules were determined under the conditions reported in European Pharmacopoeia, Ed. 7.1, paragraph 2.9.3, page 256-263 and rifaximin quantitative determination was obtained under the conditions described in European Pharmacopoeia, Ed. 7.1, 04/2011, paragraph 2362, pag. 3459.

Dissolution profiles of Normix^{®} tablets and of rifaximin tablets in gastroresistant microgranules were determined in solutions at pH 5, pH 6.5 and in FaSSIF solutions and FeSSIF solutions.

The FaSSIF solution contains taurocholate sodium 3 mM, lecithin 0.75 mM, NaH₂PO₄, 65 mM, NaCl, 85 mM, and purified water up to 1 liter, thus having a pH 6.5.

The FeSSIF solution contains taurocholate sodium 15 mM, lecithin 3.75 mM, glacial acetic acid 144.05 mM, NaCl 203.18 mM, and purified water up to 1 liter, thus having a pH 5.

The dissolution profiles reported in Table 11 were determined by using two Normix^{®} tablets and a rifaximin tablet in gastroresistant microgranules, in buffer pH 5, buffer pH 6.5, FaSSIF solution and FeSSIF solution, in a time of 360 minutes.

The dissolution profiles of the 2 of Normix^{®} tablets and of the rifaximin tablets in gastroresistant microgranules are reported in Table 11.

**Table 11**

| | **Rifaximin Normix^{®} tablets** (Rifaximin concentration µg/ml) | **Rifaximin tablets in gastroresistant microgranules** (Rifaximin concentration µg/ml) |
|---|---|---|
| Buffer pH 5 | 6.67 ± 0.29 | 4.49 ± 0.23 |
| Buffer pH 6,5 | 6.09 ± 1.01 | 9.56 ± 0.81 |
| FaSSIF | 8.40 ± 0.56 | 13.78 ± 0.64 |
| FeSSIF | 11.73 ± 1.99 | 20.58 ± 3.27 |

### Example 16

### Preparation of granules comprising rifaximin and amino acids

An amount of 200 g rifaximin, amino acids, hydroxypropyl methylcellulose, fumed silica and talc were mixed in a V mixer for 15 min at a speed of 16 rpm. The solid mixtures were loaded in a roller compactor and applied a pressure until 100 bar.

The granule composition is reported in Table 12.

**Table 12**

| **Component** | **Granule 1** **(grams)** | **Granule 2** **(grams)** | **Granule 3** **(grams)** |
|---|---|---|---|
| Rifaximin | 200 | 200 | 200 |
| Tryptophane | 195 | - | - |
| Serine | - | 75 | - |
| Histidine | - | - | 145 |
| Hydroxypropyl methylcellulose | 84.6 | 104.6 | 134.6 |
| Fumed silica | 0.4 | 0.4 | 0.4 |
| Talc | 20 | 20 | 20 |

The granules so obtained can be coated with coating film or gastroresistant film and used for suspension or tablet preparations.

### Example 17

### Preparation of granules comprising rifaximin and amino acids

An amount of 200 g rifaximin and amino acids, hydroxypropyl methylcellulose, fumed silica and talc were mixed in a V mixer for 15 min at a speed of 16 rpm. The solid mixtures were loaded in a roller compactor and applied a pressure until 100 bar.

The granule composition is reported in Table 13.

**Table 13**

| **Component** | **Granule 4** **(grams)** | **Granule 5** **(grams)** | **Granule 6** **(grams)** |
|---|---|---|---|
| Rifaximin | 200 | 200 | 200 |
| Tryptophan | 195 | 195 | 195 |
| Valine | 117 | | - |
| Leucine | - | 131 | |
| Isoleucine | | | 131 |
| Hydroxypropyl methylcellulose | 84.6 | 104.6 | 134.6 |
| Fumed silica | 0.4 | 0.4 | 0.4 |
| Talc | 20 | 20 | 20 |

The granules so obtained can be coated with coating film or gastroresistant film and used for suspension or tablet preparations.

### Example 18

### Preparation of Sachets comprising granules of amino acid and rifaximin

The granules obtained as in Example 16 were added to excipients and the resulting solid mixtures were divided in sachets. The sachet unitary composition is reported in Table 14.

**Table 14**

| **Component** | **Sachet 1** **(mg)** | **Sachet 2** **(mg)** | **Sachet 3** **(mg)** | **Sachet 4** **(mg)** | **Sachet 5** **(mg)** | **Sachet 6** **(mg)** |
|---|---|---|---|---|---|---|
| Granule 1 - *(Example 16)* | 500 | | | | | |
| Granule 2 - *(Example 16)* | | 400 | | | | |
| Granule 3 - *(Example 16)* | | | 500 | | | |
| Granule 4 - *(Example 17)* | | | | 500 | | |
| Granule 5 - *(Example 17)* | | | | | 400 | |
| Granule 6 - *(Example 17)* | | | | | | 500 |
| Hydrophobic Colloidal Silica | 10 | 10 | 10 | 10 | 10 | 10 |
| Aspartame | 20 | 20 | 20 | 20 | 20 | 20 |
| Cherry-flavour | 100 | 100 | 100 | 100 | 100 | 100 |
| Sobitol | 3370 | 3470 | 3370 | 3370 | 3470 | 3370 |
| TOT | 4000 | 4000 | 4000 | 4000 | 4000 | 4000 |

### Example 19

### Preparation of Tablets comprising rifaximin and amino acids

Rifaximin granules prepared as in Example 17 were mixed with excipients and all the component were mixed in a V binder and the obtained mixtures were compressed in a tabletting machine. The tablets were coated with film coating.

The unitary composition of tablets is reported in Table 15.

**Table 15**

| **Components** | **Tablet 1** **(mg)** | **Tablet 2** **(mg)** | **Tablet 3** **(mg)** | **Tablet 4** **(mg)** | **Tablet 5** **(mg)** | **Tablet 6** **(mg)** |
|---|---|---|---|---|---|---|
| Granule 1 - *(Example 16)* | 500 | | | | | |
| Granule 2 - *(Example 16)* | | 400 | | | | |
| Granule 3 - *(Example 16)* | | | 500 | | | |
| Granule 4 - *(Example 17)* | | | | 500 | | |
| Granule 5 - *(Example 17)* | | | | | 400 | |
| Granule 6 - *(Example 17)* | | | | | | 500 |
| Sodium starch glycolate | 15 | 15 | 15 | | | |
| Colloidal silica | 1 | 1 | 1 | | | |
| Talc | 1 | 1 | 1 | | | |
| **Coating film** | | | | | | |
| Hydroxypropyl methylcellulose | 5.15 | 5.15 | 5.15 | 5.15 | 5.15 | 5.15 |
| Titanium dioxide | 1.5 | 1.5 | 1.5 | 1.5 | 1.5 | 1.5 |
| Disodium edetate | 0.02 | 0.02 | 0.02 | 0.02 | 0.02 | 0.02 |
| Propylene glycol | 0.5 | 0.5 | 0.5 | 0.5 | 0.5 | 0.5 |
| Red iron oxide E172 | 0.15 | 0.15 | 0.15 | 0.15 | 0.15 | 0.15 |

The excipients were sieved and then mixed with rifaximin granules; the resulting mixtures were compressed using a rotary tabletting machine equipped with oblong and tablet obtained.

The tablets were coated using conventional pan equipment, in order to improve appearance and achieve taste mask properties.

The tablets can be coated also with gastroresistant film coating.

### Example 20

### Preparation of conglomerates of rifaximin and amino acids

Conglomerate A: Rifaximin - Tryptophan 1:4. A volume of 5 ml ethanol/water 1:4 (v/v) solution was e added to composition A - Example 1; the solution was then heated at 100°C until complete dissolution, and left for complete solvent evaporation at room temperature for 4 days. Rifaximin conglomerates characterized by the contemporary presence of rifaximin crystals and tryptophan crystals were obtained.

Conglomerate B: Rifaximin - Serine 1:3. A volume of 5 ml ethanol/water 1:4 (v/v) solution was added to composition B - Example 1. The solution was then heated at 100°C until complete dissolution, and left for complete solvent evaporation at room temperature for 4 days. Rifaximin conglomerate was characterized by the presence of rifaximin crystals and serine crystals.

Conglomerate C: Rifaximin- Histidine 4-1. A volume of 5 ml ethanol/water 1:4 (v/v) solution was added to composition C - Example 1. The solution was then heated at 100°C until reaching complete dissolution, and left for spontaneous evaporation at room temperature for 4 days. Rifaximin conglomerate characterized by the presence of rifaximin crystals and histidine crystals were obtained.

Conglomerate D: Rifaximin -Histidine 4:1. A volume of 5 ml ethanol/water 1:4 (v/v) solution was added to composition D - Example 1. The solution was then heated at 100°C until complete dissolution, and left for spontaneous evaporation at room temperature for 4 days. Rifaximin conglomerate characterized by the presence of rifaximin crystals and histidine crystals were obtained.

### Example 21

### Determination of water solubility of rifaximin conglomerates

The solubility of conglomerates A, obtained according to Example 20, was determined by placing 653 mg of this solid in 5 ml phosphate buffer at pH 6.8 at room temperature and the solution was left for 24 under stirring.

Rifaximin concentration in solution was determined by spectrophotometric method at wavelength 364 nm. The rifaximin concentration was 28 µg/ml.

## Claims

1. Rifaximin crystals **characterized in that** they are obtained by means of a process comprising:
a) dissolution of the compositions of rifaximin and amino acids, wherein amino acids and rifaximin are in a molar ratio comprised between 1:1 and 10:1, in solutions formed by ethanol/water, in a volumetric ratio comprised between 1:1 and 1:10 (v/v);
b) evaporation of the solution obtained in step a) at temperatures comprised between room temperature and 40°C, in a time period comprised between 1 and 10 days;
wherein the resulting crystals have monoclinic space group *P*2₁ and cell parameters comprised in the ranges:
a: 13.7(1)-13.8(1) *Å;* b: 19.7(1)-19.9 *(1)Å;* c: 16.4(6)-16.6(6) *Å;* β: 92.1(1)-91.9(1) deg.

2. Rifaximin crystals according to claim 1, **characterized in that** they have 3 water molecules for each rifaximin molecule.

3. Rifaximin crystals according to claim 1, **characterized in that** they have 4.5 water molecules for each rifaximin molecule.

4. Rifaximin crystals **characterized in that** they are obtained by means of a dehydration process of the crystals obtained according to claim 1, in the presence of dehydrating agents, wherein the resulting crystals have monoclinic space group *P*2₁ and cell parameters comprised in the ranges:
a: 14.2(1)-14.5(1) *Å* ; b: 19.7(1)-20.1(1) *Å;* c: 16.1(1)-16.2(1) *Å;* β: 108.7(1)-111.4(1) deg.

5. Rifaximin crystals according to claim 4, **characterized in that** they have zero water molecules for each rifaximin molecule.

6. Rifaximin crystals according to claim 4, **characterized in that** they have 0.5 water molecules for each rifaximin molecule.

7. Rifaximin crystals according to claim 4, **characterized in that** they have 1.5 water molecules for each rifaximin molecule.

8. Process for the production of rifaximin crystals, **characterized in that** it comprises:
a) dissolution of the compositions of rifaximin and amino acids, wherein amino acids and rifaximin are in a molar ratio comprised between 1:1 and 10:1, in solutions formed by ethanol/water, in a volumetric ratio comprised between 1:1 and 1:10 (v/v);
b) evaporation of the solution obtained in step a) at temperatures comprised between room temperature and 40°C, in a time period comprised between 1 and 10 days;
wherein the resulting crystals are **characterized by** monoclinic space group *P*2₁ and cell parameters comprised in the ranges:
a: 13.7(1)-13.8(1) *Å;* b: 19.7(1)-19.9 *(1)Å;* c: 16.4(6)-16.6(6) *Å;* β: 92.1(1)-91.9(1) deg.

9. Process for the production of rifaximin crystals, **characterized in that** it comprises:
a) dissolution of the compositions of rifaximin and amino acids, wherein amino acids and rifaximin are in a molar ratio comprised between 1:1 and 10:1, in solutions formed by ethanol/water, in a volumetric ratio comprised between 1:1 and 1:10 (v/v);
b) evaporation of the solution obtained in step a) at temperatures comprised between room temperature and 40°C, in a time period comprised between 1 and 10 days, in the presence of dehydrating agents;
wherein the resulting crystals have monoclinic space group *P*2₁ and cell parameters comprised in the ranges:
a: 14.2(1)-14.5(1) *Å* ; b: 19.7(1)-20.1(1) *Å;* c: 16.1(1)-16.2(1) *Å;* β: 108.7(1)-111.4(1) deg.

10. Use of amino acids and rifaximin in a molar ratio comprised between 1:1 and 10:1 in order to obtain rifaximin crystals, **characterized by** monoclinic space group *P*2₁ and cell parameters comprised in the ranges:
a: 13.7(1)-13.8(1) *Å;* b: 19.7(1)-19.9 *(1)Å;* c: 16.4(6)-16.6(6) *Å;* β: 92.1(1)-91.9(1) deg.

11. Use of amino acids and rifaximin in a molar ratio comprised between 1:1 and 10:1 in order to obtain rifaximin crystals, **characterized by** monoclinic space group *P*2₁ and cell parameters comprised in the ranges:
a: 14.2(1)-14.5(1) *Å* ; b: 19.7(1)-20.1(1) *Å;* c: 16.1(1)-16.2(1) *Å;* β: 108.7(1)-111.4(1) deg.
